# EUROPEAN PATENT SPECIFICATION

(11) **EP 2 696 752 B1**
(45) Date of publication and mention of the grant of the patent: **04.12.2019**
(21) Application number: 12715908.5
(22) Date of filing: 11.04.2012
(51) Int. Cl.: A61B 5/0408, H01B 1/24, A41D 13/12, A61F 13/00, D03D 15/00

(54) **FABRIC FOR ACQUIRING PHYSIOLOGICAL SIGNALS**
SCHALTSTOFF ZUR ERFASSUNG PHYSIOLOGISCHER SIGNALE
TISSU SERVANT À ACQUÉRIR DES SIGNAUX PHYSIOLOGIQUES

(30) Priority: 12.04.2011 EP 11162135; 12.04.2011 US 201161474484 P
(43) Date of publication of application: 19.02.2014
(73) Proprietor: Smart Solutions Technologies, S.L., 28290 Las Matas (Madrid) (ES)
(72) Inventor: MACIÁ BARBER, Agustin, 28250 Torrelodones (Madrid) (ES); LLORCA JUAN, Daniel, 63017 Porto San Giorgio (IT); VICENTE RENGEL, Christian, 46200 Paiporta (Valencia) (ES); GONZÁLVEZ MUÑOZ, Borja, 28290 Las Matas (Madrid) (ES)
(74) Representative: Grund Intellectual Property Group Patentanwalt und Solicitor PartG mbB
(86) International application number: PCT/EP2012/056573
(87) International publication number: WO 2012/140079

(56) References cited:
- EP-A1- 2 196 142
- WO-A1-97/18450
- WO-A1-2004/058346
- US-A- 5 164 443
- US-A1- 2008 242 176
- ANJUM SALEEM ET AL: "Fabrication of Extrinsically Conductive Silicone Rubbers with High Elasticity and Analysis of Their Mechanical and Electrical Characteristics", POLYMERS, vol. 2, no. 3, 10 August 2010 (2010-08-10) , pages 200-210, XP055007217, DOI: 10.3390/polym2030200
- CARPI F ET AL: "Electroactive Polymer-Based Devices for e-Textiles in Biomedicine", IEEE TRANSACTIONS ON INFORMATION TECHNOLOGY IN BIOMEDICINE, IEEE SERVICE CENTER, LOS ALAMITOS, CA, US, vol. 9, no. 3, 1 September 2005 (2005-09-01), pages 295-318, XP011138577, ISSN: 1089-7771, DOI: 10.1109/TITB.2005.854514

## Description

### FIELD OF THE INVENTION

The present invention relates to a fabric which comprises at least an elastic and electrically conductive area integrated into the fabric, a process to obtain the fabric, as well as to the use of a silicone rubber, loaded with an electrically conductive material, for the preparation of the fabric of the invention. It also relates to a device comprising the fabric, as well as a garment comprising the device.

### BACKGROUND ART

It is known in the state of the art, fabrics which comprises electrodes and tracks enable the realization of wearable garments, capable of recording physiological signals and to be used during everyday activities. The electrodes are placed in contact with the skin of the human body and the electrical physiological signals which result are examined. Electrocardiograms (ECG) or electromyograms (EMG) are examples of physiological signals to be monitored through the garment.

Nevertheless, stability, noise and sensibility of the signals can be affected by different reasons; motion and long term acquisition of the signal are two of the most significant.

The electrode and track to be integrated in a garment must be a system minimal invasive, flexible, conformable to the human body including in movement and resistant to repeated washing.

To reduce the noise the traditional electrodes use adhesive to attach the electrode to the skin. The scope for electrodes integrated in fabrics is to eliminate the adhesive using the fabric's pressure to the body. In order to make pressure, the fabrics are flexible and elastic being able to adapt to every different type of body. If the wiring system is not elastic every movement made by the body will be translated into the electrode moving it from its place, the elastic circuit works as a spring between the electrode and connector. That is the reason why the need to find and elastic and no directional wiring system integrated in fabrics.

The document Anjum Saleem et al., "Fabrication of extrinsically conductive silicone rubbers with high elasticity and analysis of their mechanical and electrical characteristics", Polymers 2010, vol. 2 (3), pp. 200-210, describes the need and efforts to find a silicone rubber reinforced with conductive filler that allows this functionality, and the document presents the difficulties to achieve it. Mix silicon rubber with conductive filler until the percolation threshold it doesn't seam enough to obtain conductivity, only the carbon fibers are able to achieve a semi conductive value. The issue with carbon fibers is that they reduce the silicon mechanical properties, so they are not suitable for high elastic products.

The use of silicon conductive rubber on fabrics is described in different patents but as the publications explain the use of room cure temperature conductive silicone rubber to print directly into fabrics is still not solve.

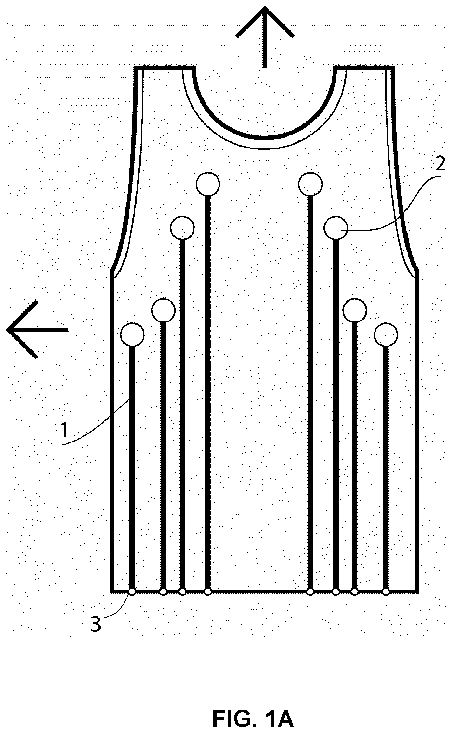

The current state of the art in fabric for acquiring physiological signals presents different drawbacks for example, the patent publication US7779656, which applicant is SmartLife Technology Limited, describes knitting techniques, particularly techniques applied to garment in which the yarns have conductive characteristics. Such garments are useful for monitoring physiological signals of the wearer. The electrodes are attached to or incorporated in the garment, the track are conductive yarns connected to a terminal connector located elsewhere on the garment. The tracks have two directions possible to be integrated in the fabric so the track has serious limitations to connect the electrode and the terminal connector when the electrodes are located in different places. This situation is represented in the FIG. 1A of the present document.

The patent publication US7783334, which applicant is Electronics and Telecommunications Research Institute, describes a garment for measuring physiological signals comprising an electrode which is made of an electro conductive fabric and detects a physiological signal; a track through which the detected physiological signal is transmitted, a physiological signal measuring unit which is connected to the transmission line, receives the physiological signal, and measures information regarding body conditions related to the physiological signal, and a pocket where the physiological signal measuring unit in inserted. The tracks, made of an electro conductive thread, are not integrated in the garment, they are fixed to the garment by a tuck. This option has some limitations, the no integration of the track in the fabric means that the garment is not comfortable on the other hand the elasticity of the tracks are low because they are made of metal threads.

The patent application US2010198038 describes a electrode sheet which includes: a material with a flattened surface; a wiring layer provided on the flattened surface of the material and formed of a conductive ink containing carbon nanotubes; and an electrode connected to the wiring layer. The conductive ink is not elastic. The composition of the conductive inks is completely different of the composition of a silicone rubber. It is necessary applied the inks in a flattened area. When the area is a textil it is necessary to apply a first layer because the weave of the textil present orificies The biggest issue with conductive inks is the mechanical properties, since the fabric must support water, abbrasion, strech, impacts, etc. The current conductive inks don't fit this requirements. The description of the material is not just important for the conductive properties but for the mechanical properties.

US 2008/242176 relates to a heat resistance fabric impregnated with polysiloxane. US 5164443 relates to an electroconductive silicone composition containing acetylene black as a carbon black. WO 2004/058346 describes a fabric based electrode comprising an electrically conductive material attached to an electrically conductive electrode portion. WO 97/18450 describes a sock containing piezoresistive pressure sensors encapsulated in a thin, flexible polymer sandwich. Carpi *et al.* relates generally to electroactive polymer-based devices for e-textiles in biomedicine.

Thus, from what is known in the art, it is derived that the development of a fabric which comprises an elastic electrically conductive area integrated into the fabric, that may be a track is still of great interest.

### SUMMARY OF THE INVENTION

In the acquisition of some physiological signals, as the ECG, is important to obtain a multiple signals in order to give an accurate diagnostic. The quantity of electrodes in a fabric for acquiring ECG signals is limited to the possibility to connect the electrode to the device that collecting the signals via the tracks. A track made in a flexible, elastic and conductive material is a goal to connect as many electrodes as been necessary.

The location and quantity of electrodes may modify the garment performance due to track limitations, in order to don't decrease the fabric performance the track must be integrated into the fabric.

In the acquisition of some physiological signals, as the ECG, is important to guaranty the electrode adherence to the body, being integrated in a fabric the electrode doesn't contain any adhesive to attach to the skin. For that purpose reduce the movement of the electrode in activity is extremely necessary to compete to the signal obtain by traditional electrodes.

In a first aspect of the present invention, it is provided a fabric with at least an elastic and electrically conductive track having a thickness of from 120 µm to 800 µm wherein the elastic and electrically conductive track is integrated into the fabric, characterized in that the elastic and electrically conductive track consists of a silicone rubber loaded with an electrically conductive material which has penetrated into interstices between strands of the fabric thereby integrating and anchoring the elastic and electrically conductive track into the fabric. The elastic and electrically conductive area is a track which can be connected to an electrode for acquiring physiological signals, e.g. ECG signals. The electrically conductive area may have any shape and any direction. This opens the possibility to place as many electrodes as been necessary into the fabric, and then connect all the electrodes via the electrically conductive areas to an electrical connector. Each electrically conductive area works like an independently track.

Therefore, according to the present invention, the terms "elastic and electrically conductive area" and "elastic and electrically conductive track" are each interchangeable.

The fabric with an electrically conductive area that comprises a silicone rubber loaded with an electrically conductive material improves the flexibility, the elasticity and the conductivity of the track. The flexibility and the elasticity of the silicone rubber loaded with an electrically conductive material allow that conductivity is not interrupted with the movement of the fabric. Furthermore the silicone doesn't lose its properties with the washing.

Nothing in the art suggests that a fabric comprising an elastic and electrically conductive area integrated into the fabric could confer excellent flexibility and elasticity properties and an excellent integration fabric-electrically conductive area.

The amount of conductive material is not important once you reach the percolation threshold, this value depends on the conductive filler and the silicone. An objective of the present invention is to be able to achieve and preserve low resistive values in an elastic fabric with silicone conductive rubber being able to apply the conductive silicone in any shape and direction.

Therefore, an aspect of the present invention relates to a fabric which comprises at least one electrically conductive area integrated into the fabric, wherein the electrically conductive area comprises a first layer of silicone rubber loaded with an amount comprised of from 5% w/w to 40% w/w of an electrically conductive material.

Prefereably at least one conductive area means one to twenty electrically conductive areas. More preferably one to ten electrically conductive areas. More preferably one, two, three, four, five, six, seven, eight, nine electrically conductive areas.

The silicone rubber loaded with an electrically conductive material of the present invention, is a room temperature curing silicone.

A room temperature curing silicone is a silicone that vulcanizes or cures at room temperature by a chemical reaction.

The conductive property of the silicone rubber depends on how close the conductive material are, e.g. carbon fibers (CF), carbon black (CP), nickel coated graphite (NG), copper fibers (Cu). As the publication of Anjum Saleem et al. above mentioned describes, it is important to reach at least the quantity of conductive material, e.g. CF, to meet the percolation threshold. As this point depends on the number and distance between the carbon fibers (CF) its obvious that when we stretch the material will go underneath the percolation threshold, in order to assure the conductivity during the stretch the material has to go beyond the percolation threshold and get as closer as it can to the saturation point, but as we get closer to the saturation point the silicone will lose mechanical properties. As the publication presents the right mix is not enough to obtain low resistive values.

The process for the preparation of the elastic and electrically conductive area integrated into the fabric comprises a step of applying pressure when applying the silicone rubber directly to the fabric, in order to eliminate any air bubble that will break the conductivity. For this purpose the application system is a screen-printing process using low speed and high pressure. The pressure to be applied is comprised of from 0.2 to 0.8 Kg/m², preferably comprised of from 0.3 to 0.5 Kg/m², being particularly preferred a pressure of 0.45 Kg/m².

The low speed will allow the high-viscosity silicone rubber to fall into the fabric at the same point the high pressure goes. An important aspect is the thickness of the coating, thicker is the conductive silicone coating better are the mechanical properties so better are the conductive properties when we stretch the material. According to an embodiment of the present invention, the elastic and electrically conductive track is at least 120 µm thick, preferably at least 200 µm thick. In accordance with a particular embodiment of the present invention, the thickness of the track is comprised of from 120-800 µm thick, preferably comprised of from 120-500 µm thick, more preferably comprised of from 250-500 µm thick, being particularly preferred comprised of from 300-400 µm thick.

The process for the preparation of the invention comprises a step of cure the silicone rubber at room temperature. In the present invention the silicone rubber is cured into de fabric. When it is required to decrease the time of cured, a step of pre-cured comprised of from 80°C to 200°C is included. Preferably, the pre-cured step is carried out at a temperature comprised of from 90°C to 165°C.

Therefore, another aspect of the invention relates to a process for the preparation of the fabric of the invention, which comprises the steps of:
a) liquid-printing a first layer of silicone rubber loaded with an amount comprised of from 5% w/w to 40% w/w of a electrically conductive material into the fabric;
b) pre-curing the first layer for up one minute at a temperature comprised of from 80°C to 200°C;
c) curing the first layer at room temperature.

Another aspect of the invention relates to the use of a silicone rubber loaded with an amount comprised of from 5% w/w to 40% w/w of an electrically conductive material for the preparation of the fabric of the invention.

The fabric of the invention comprising the elastic and conductive area may be integrated in a device for receiving and collecting and/or storing and/or processing, and/or transmitting data from said fabric.

Therefore, in another aspect of the invention is provided a device comprising:
a) the fabric of the invention,
b) an electronic instrument for receiving and collecting and/or storing and/or processing, and/or transmitting data from said fabric.

With the fabric of the invention is possible to tailor a garment.

Therefore, another aspect of the invention relates to a garment comprising the device of the invention.

### BRIEF DESCRIPTION OF THE DRAWINGS

FIG. 1A illustrates an elevation view of a garment according to the state of the art.

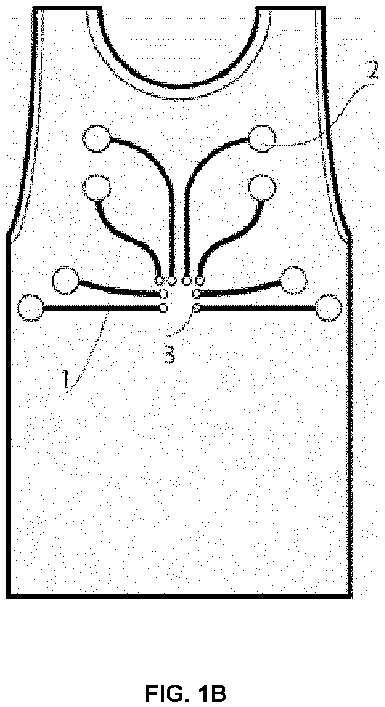

FIG. 1B illustrates an elevation view of the garment of the invention.

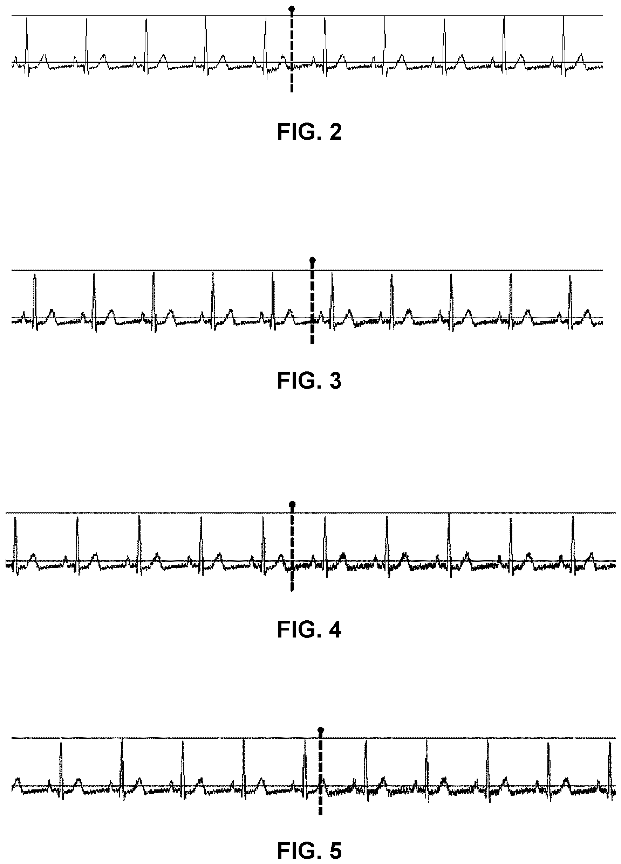

FIG. 2 shows and ECG strip where the electrically conductive area was stretched by about 25% of their original length. Left part of the strip (left of the line), the electrically conductive areas aren't stretched, and the right part of the strip (right of the line) the electrically conductive areas are 25% stretched.
FIG. 3 shows and ECG strip where the electrically conductive area was stretched by about 25% of their original length. Left part of the strip (left of the line), the electrically conductive areas aren't stretched, and the right part of the strip (right of the line) the electrically conductive areas are 25% stretched.
FIG. 4 shows and ECG strip where the electrically conductive area was stretched by about 50% of their original length. Left part of the strip (left of the line), the electrically conductive areas aren't stretched, and the right part of the strip (right of the line) the electrically conductive areas are 50% stretched. FIG. 5 shows and ECG strip where the electrically conductive area was stretched by about 50% of their original length. Left part of the strip (left of the line), the tracks aren't stretched, and the right part of the strip (right of the line) the electrically conductive areas are 50% stretched.

### DETAILED DESCRIPTION OF THE INVENTION

As mentioned above, a first aspect of the invention relates to a fabric which comprises at least an elastic and electrically conductive track having a thickness of from 120 µm to 800 µm (1) wherein the elastic and electrically conductive track is integrated directly into the fabric, and wherein the elastic and electrically conductive track comprises a silicone rubber loaded with an electrically conductive material. The fabric is able to stretch between 1 % and 200%.

The skilled person in the art knows several fabrics adequate for the invention, preferably elastic fabric, for example polyester, nylon. Non limiting example of elastic fabric is a fabric which comprises a percentage of elastane, from 3% w/w to 20% w/w.

The flexibility and the elasticity of the silicone enables the electrically conductive track 1 to be held in very good conformity and the conductivity is not interrupted.

As mentioned above the electrically conductive area 1 integrated into the fabric may works as a track. Thus, in a particular embodiment of the present invention, the fabric comprises at least an electrically conductive area 1 (track), at least an electrode 2 electrically in contact with the track 1, and at least an electrical connector 3 placed in the track 1. Therefore, the electrically conductive area 1, track, transmits an electrical signal from an electrode 2 placed in contact with the skin of a user to an electrical connector 3 placed in the electrically conductive area 1, the track. The connector 3 may be in contact with an electronic instrument for receiving and collecting and/or storing and/or processing, and/or transmitting data from said fabric.

The electrically conductive area described herein, comprises a first layer of silicone rubber loaded with an electrically conductive material. The silicone rubber before the process of curing is in a liquid high-viscosity state. When the silicone is in the high-viscosity state is printed in the fabric. This means that the union silicone-fabric is an union without an adhesive. The silicone in the high-viscosity state when is printed in the fabric is capable to penetrate in the orifices of the fabric, anchored with the structure of the fibers of the fabric. Therefore, the electrically conductive area described in the present invention is integrated into the fabric.

Accordingly, it is also encompassed into the scope of the present invention a fabric which comprises at least an elastic and electrically conductive track integrated into the fabric, wherein the elastic and electrically conductive track comprises a silicone rubber loaded with a electrically conductive material, obtainable by the steps:
a) screen-printing, applying a pressure comprised of from 0.2 to 0.8 Kg/m², a first coating of silicone rubber loaded with a electrically conductive material on the fabric;
b) pre-curing the first coating for up one minute at a temperature of at between 80°C to 200°C;
c) curing the first coating at room temperature; being the thickness of the printed coating comprised of from 120 to 800 µm thick.

The electrically conductive material which are added to silicone rubber for imparting electric conductivity is selected from carbon black, graphite or various metal powders such as silver, nickel, and copper. Preferably, the electrically conductive material is carbon black.

The term "carbon black" as used herein, refers to carbon in the form of colloidal particles that are produced by incomplete combustion or thermal decomposition of gaseous or liquid hydrocarbons under controlled conditions. Its physical appearance is that of a black, finely divided pellet or powder. There are different types of carbon black in relation with the reaction condition, these are for example furnace black, lamp black, thermal black, acetylene black, channel black.

In a preferred embodiment the percentage of the conductive material is between 10% to 35%. In a more preferred embodiment the percentage of the conductive material is between 15% to 30%. In another preferred embodiment the percentage of the conductive material is between 20% to 25%.

In another embodiment of this aspect of the invention the fabric further comprises a coating of an insulating material covering the layer of silicone rubber loaded with an electrically conductive material 1. An example of insulating material is an anti-slip silicone; this silicone has a material/skin friction coefficient of al least 0.5.

The fabric of the invention may acquires physiological signal when comprises an electrode 2 to be placed in contact with the skin.

Therefore, in another embodiment of this aspect of the invention the fabric comprises an electrode 2 to be placed in contact with the skin of an user, in electrical contact with the electrically conductive area 1.

The term "electrode" as used herein, refers to the area of conductive layer that is in contact with the skin and wherein a physiological signal is received or an electrical impulse is transmitted to the user.

In a preferred embodiment of this aspect the electrode 2 comprises a conductive fabric made of conductive fibers and non conductive fibers. More preferably the electrode 2 refers to a conductive fabric made of conductive fibers.

Preferably, the conductive fibers are made of silver coated nylon (such as X-static® yarns from Laird Sauquoit Industries) and the non conductive fibers are made of nylon. Non limiting examples of conductive fibers are fibers made of silver, copper, nickel, stainless steel, gold, non conductive fibers coated with a conductive material or mixtures thereof. Non limiting examples of non conductive fibers are wool, silk, cotton, flax, jute, acrylic fiber, polyamide polyester, nylon and/or with elastic yarns (such as LYCRA® branded spandex from Invista™ S.a.r.l).

In a preferred embodiment of this aspect the electrode 2 is a layer of silicone rubber loaded with an amount between 5% w/w to 40% w/w of an electrically conductive material, which is integrated into the fabric. When the flexible, elastic and conductive electrode is elongate, the fabric support extends substantially the full length of that layer. The flexibility and elasticity of the silicone enables the electrode to be held in very good conformity and electrical surface-contact with the patient's skin throughout substantially the whole area.

In the electrocardiogram (ECG) measurement, the contact resistance between the skin of a human body and the electrodes can be about several MΩ. Thus, a resistance value, from one end (electrode contact portion) of the silicone rubber loaded with electrically conductive material to the other end (connector portion), of 1000 KΩ or less is sufficient for practical use when the silicone rubber loaded with electrically conductive material is stretched by about 50%.

Therefore in a embodiment of this aspect the electrical resistance per cm of the silicone rubber loaded with an electrically conductive material is 1000 KΩ/cm or less, preferably 500 KΩ/cm or less. In another embodiment of this aspect of the invetion the electrical resistance per cm of the silicone rubber loaded with an electrically conductive material is comprised of from 50 Ω/cm to 100 kΩ/cm, preferably comprised of from 1 KΩ/cm to 100 KΩ/cm, particularly preferred the resistance per cm value is comprised of from 50 Ω/cm to 10 KΩ/cm.

In another embodiment of this aspect the cured temperature of the silicone rubber loaded with an electrically conductive material is between 20°C to 200 °C. A more preferred embodiment the cured temperature is between 50 °C to 140 °C. In another preferred embodiment the cured temperature is between 100 °C to 120 °C.

The silicone rubber loaded with an electrically conductive material contains a platinum catalyst, diorganopolysiloxane having silicon-bonded alkenyl groups, organohydrogenpolysiloxane and an electrically conductive material.

Therefore in a embodiment of this aspect the silicone rubber loaded with an amount between 5% w/w to 40% w/w of a electrically conductive material comprises:
a) diorganopolysiloxane having silicon-bonded alkenyl groups;
b) organohydrogenpolysiloxanes;
c) a platinum catalyst; and
d) an electrically conductive material.

Examples of the diorganopolysiloxane having silicon-bonded alkenyl groups are dimethylvinylsiloxy- terminated dimethylpolysiloxane gums, dimethylallylsiloxy-terminated dimethylpolysiloxane gums, phenylmethylvinylsiloxy-terminated diphenylsiloxane-dimethylsiloxane copolymer gums, dimethylvinylsiloxy-terminated methylvinylsiloxane-dimethylsiloxane copolymer gums and silanol-terminated methylvinylsiloxane-dimethylsiloxane copolymer gums.

Examples of the organohydrogenpolysiloxanes are trimethylsiloxy-terminated methylhydrogenpolysiloxanes, trimethylsiloxy-terminated dimethylsiloxanemethylhydrogensiloxane copolymers,dimethylphenylsioxy-terminated methylphenylsiloxanemethylhydrogensiloxane copolymers, cyclic methylhydrogenpolysiloxanes and copolymers composed of dimethylhydrogensiloxy units and SiO₄/₂ units.

Several platinum catalyst are known as curing acceleration catalysts for silicone compositions which cure by a hydrosilation reaction. Example of the platinum catalysts are: platinum black, platinum on active carbon, platinum on silica micropowder, chloroplatinic acid, alcohol solutions of chloroplatinic acid, platinum olefin complexes, platinum tetrachloride, platinum vinylsiloxane complexes, chloroplatinic acid-olefin complexes, chloroplatinic acid methylvinylsiloxane complexes.

In a preferred embodiment of the this aspect the silicone rubber loaded with an amount between 5% w/w to 40% w/w of a electrically conductive material comprises:
a) divinylpolydimethylsiloxane in a percentage between 60% w/w to 75% w/w;
b) dioxosilane in a percentage between 7% w/w to 15% w/w,
c) carbon black in a percentage between 5% w/w to 15% w/w,
d) platinum (0)-1,3-divinyl-1,1,3,3-tetramethyl disiloxane (CAS No. 68478-92-2) in a percentage between 0,001% w/w to 0,05% w/w and;
e) polydimethylthydrogensiloxane in a percentage between 3% w/w to 7% w/w.

The high degree of adhesion strengh between the fabric and the elastic and electrically conductive material is achieve because the coating material can readily penetrate the interstices between the strands anchoring with the structure of the fibers of the fabric, resulting in the integration of the elastic and electrically conductive material into the fabric.

Liquid-printing is a coating method wich combines laminating and liquid coating, in this case the silicone to be coated is a liquid (high viscosity) silicone but instead of be applied in both sides is applied just in one side of the fabric similar as the laminating process. The thickness control is important in coatings because they change the fabric properties in mesure of the thickness of the coating.

As is well known in the art, the term liquid-printing encompasses a family of printing processes where the printed material in liquid state is deposited on the support. Within this family of processes are: screen-printing and digital-printing. In the digital-printing process the material is directly applied by a dispenser that reproduces the digitally processed design. Screen-printing process the liquid material is deposited using a stencil. The stencil can be made in different design and thickness.

As mentioned above in another aspect of the invention is provided a preparation process of the fabric of the invention, which comprises the steps of:
a) liquid-printing a first coating of silicone rubber loaded with an amount between 5% w/w to 40% w/w of a electrically conductive material on the fabric;
b) pre-curing the first coating for up one minute at a temperature of at between 80°C to 200°C;
c) curing the first coating at room temperature.

According to an embodiment of the present invention, the liquid-printing process is a screen-printing process.

The term "room temperature" as used in the present invention, refers to a temperature between 20°C to 30°C for example 25°C.

A printed circuit board is a conductive wiring system coated in the board by printing the conductive material on the board, different electrical components can be bond to the conductive wiring system in order to achieve different purposes. The present invention describes a circuit with elastic and flexibility mechanical properties, where the board is fabric mesh and the wiring system is conductive silicone printed on the fabric. Once the silicone is cured is not possible to connect any electronic component, so in order to use this silicone as wiring system the electronic components will be place in the fabric before applying the liquid conductive silicone, this method is described as the preferred embodiment comprising the following steps:
a) coating the electrode with a thermal adhesive
b) fixing the electrode to the fabric;
c) liquid-printing a first layer of silicone rubber loaded with an amount between 5% w/w to 40% w/w of a electrically conductive material on the fabric;
d) pre-curing the first layer for up one minute at a temperature of at between 80°C to 200°C;
e) coating a layer of an insulating material covering the first layer of the silicone rubber loaded with an electrically conductive material;
f) curing at room temperature;
g) putting the connector.

In a preferred embodiment, the first layer of silicone rubber loaded with the electrically conductive material is screen-printed with a thickness comprised of from 120-800 µm, preferably comprised of from 200-500 µm, being particularly preferred comprised of from 300-400 µm.

The electrode is placed in the fabric in such a way that it is electrically in contact with the track.

The steps a) and b) describes the process for the preparation of the electrode, the steps c) to f) describes the process for the preparation of the electrically conductive area. The process for the preparation of the electrically conductive area, steps c) to g) can be carried out before the process for the preparation of the electrode steps a) and b).

When the fabric further comprises a second silicone rubber layer placed between the fabric and the first layer of silicone rubber loaded with an electrical conductive material before the step d), a step of liquid printing the silicone and a step of pre-cured the second silicone can be carried out.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

### Example 1.

It was measured the performance of the fabric of the invention with different levels of streching to evaluate how it may affect the quality of the signal.

The fabric in the example comprises a electrically conductive area which comprises a conductive silicone (VP97065/30 from Alpina Technische Produkte GmbH), two electrodes of conductive fabric made of conductive fibers and non conductive fibers, the conductive fibers are made of silver coated nylon (X-static® yarns from Laird Sauquoit Industries) and non conductive fibers are made of nylon.

To test and evaluate the signals transmitted through the electrically area which comprises the conductive silicone VP97065/30, it was followed a test in which the electrically conductive area was subjected to different levels of stretching to evaluate how much the signal is distorted.

Three states are evaluated: resting, electrically conductive area stretched by about 25% and electrically conductive area stretched by about 50%.

The signal was generated by a PS420 Multiparameter Patient ECG Simulator (from Fluke Corporation) and passed through electrodes, and the signal is conducted via the electrically area which comprises the conductive silicone to an electronic instrument for receiving and transmitting the signal to a computer for visualization and further analysis.

The levels of electrically conductive area streching were the following
Resting: the electrically conductive area is not stretched, keeping their original length 6.5 cm.
25% Stretching: the electrically conductive area stretched by about 25%
of their original length, 8.125 cm.
50% Streching: the electrically conductive area stretched by about 50% of their original length, 9.75 cm.

For each state (resting, 25% and 50% stretching) it was captured two segments of signal that consists in 9-10 heart beats of the ECG Simulator (10 seconds each segment because the simulator is configured at 60 beats per minute).

When the different electrocardiographic signals were obtained with the different levels of stretching we make a sort of measures over these signals to evaluate the performance the electrically area which comprises the conductive silicone. These were the measures performed on the signals:

### Visual Measures

This measure was a direct recognition, just by watching the signal, of the quality of the signal acquired in terms of morphology and noise detected. This visual recognition is also used to identify what beats (QRS complexes) and characteristic waves were recognizable and which of them are too noisy to be recognized by a cardiologist. A total of 500 beats were analyzed for each different level of electrically conductive area streching.

### Measures over the signal

These measures were made on the signal registered in each level of stretching. These measures involve manual and automatic analysis of the recorded signals.

Cross correlation: the signal was separated between the different levels of stretching and compared with the correlation between each other. The cross-correlation was a measure of similarity of two waveforms as a function of a time-lag applied to one of them. This was very helpful because it was used an ECG Simulator that always generates the same beats with no difference between them. That means that if we do the cross-correlation between two signals (one with no stretching and one with stretching), the only difference between them will be the noise. This measure goes from 0 (no similarity, completely different) and 1 (the signals are equal).

RMS Noise: the RMS (Root Mean Square) of the T-P segment was be calculated in between heartbeats. This measure was done for each stretching level and, averaged, will give us an estimate of the noise in the signal. These measures were done manually (to select the beginning and end of each segment).

Both values were very important and very good estimators of the noise present in the signal and the distortion introduced by the stretching of the silicone rubber loaded with electrically conductive material.

### Results

This section presents the results obtained by the test protocol followed. These results involve all the measures taken that have been described in the previous sections. The information was presented divided in two sections: Visual Results and Measures over the signal.

### Visual Results obtained taking captures of the signal directly from the computer

The line that crosses the ECG strips indicates the point where the stretching started and maintained until the end of the strip.

### a) 25% Streching

Two examples, (FIG. 2, FIG: 3), left part of the strip (left of the line), the electrically conductive areas aren't stretched, and the right part of the strip (right of the line) the electrically conductive areas are 25% stretched.

### b) 50% Streching

Two examples, (FIG. 4, FIG. 5), left part of the strip (left of the line), the electrically conductive areas aren't stretched, and the right part of the strip (right of the line) the electrically conductive areas are 50% stretched.

From these signals was easily observed that the quality of the signal is barely affected by the stretching of the electrically conductive area . More noise was present and visible when the track is stretched a 50%, but this noise was not enough to corrupt the signal and all the waves and characteristic points were still visible, and also was a noise easily filtered in a post processing.

### Signal Measures Results

In this section it is showed the results obtained with the measures made, manually and automatically, over the signal as was explained before. These results give a more accurate approach of the noise and quality of the signal.

### a) RMS Noise

### 25% Streching

The results are given for four different segments, two of them with the electrically conductive area not stretched (NO STRETCH_1 and NO STRETCH_2) and the other two with the electrically conductive areas 25% stretched (25% STRETCHING_1 and 25% STRETCHING_2).

**Table 1. RMS Noise**

| | RMS Noise |
|---|---|
| No Stretch 1 | 0.11918993 |
| 25% Streching 1 | 0.13268027 |
| No Stretch 2 | 0.14075932 |
| 25% Streching 2 | 0.14376695 |

In both cases, the signal without stretching the electrically conductive areas had less noise than when the electrically conductive area was stretched after that. It is clearer with average results.

**Table 2. Average RMS Noise**

| | RMS Noise |
|---|---|
| No Stretch | 0,12997463 |
| 25% Streching | 0,13822361 |

### 50% Streching

The results are given for four different segments, two of them with the electrically conductive area not stretched (NO STRETCH_1 and NO STRETCH_2) and the other two with the electrically conductive area 50% stretched (50% STRETCHING_1 and 50% STRETCHING_2).

**Table 3. RMS Noise**

| | RMS Noise |
|---|---|
| No Stretch 1 | 0,14470239 |
| 50% Streching 1 | 0,14615933 |
| No Stretch 2 | 0,14576144 |
| 50% Streching 2 | 0,15123728 |

In both cases, the signal without stretching the electrically conductive area had less noise than when the electrically conductive area was stretched after that. It is clearer with average results.

**Table 4. Average RMS Noise**

| | RMS Noise |
|---|---|
| No Stretch | 0,14523191 |
| 50% Streching | 0,1486983 |

It was also very significant that the difference between the two states were very low, so that indicates that very little noise is present due to the stretching of the electrically conductive areas.

### Cross correlation

This measure gives an estimate of the signal quality, being values next to one the best ones because that indicates that the signal captured with the conductive not stretched is equal to the signal captured with the electrically conductive area stretched. The next Table 5 shows the results for the 25% Stretching and 50% Stretching.

**Table 5. Cross Correlation**

| | Cross Correlation |
|---|---|
| No Stretch/25% Strech | 0.975041781 |
| No Stretch/50% Strech | 0.960290 |

Table 5 shows that the signal was barely corrupted by noise in both situations. As expected, the 50% stretching was a little bit worse but the results were extremely good with a similarity of 96% (only 4% of noise) in the worst case.

Analyzing the data shown here, it has been extracted some conclusions that define the performance of the conductive electrically conductive area : the electrically conductive area are appropriate to transmit bio-potentials, and in particular as seen here, electrocardiographic signals, the electrically conductive area behave very well in situations of stretching, with very little corruption (noise) of the signal, all the characteristic points and waves of the electrocardiogram are perfectly recognized when the electrically conductive area was stretched, so there were no problems with visual inspection of the ECG.

### REFERENCES CITED IN THE APPLICATION

US7779656

US7783334

US2010198038

Anjum Saleem et al., "Fabrication of extrinsically conductive silicone rubbers with high elasticity and analysis of their mechanical and electrical characteristics", Polymers 2010, vol. 2 (3), pp. 200-210

## Claims

1. A fabric for acquiring physiological signals comprising at least an elastic and electrically conductive track having a thickness of from 120 µm to 800 µm (1) wherein the elastic and electrically conductive track is integrated into the fabric, **characterised in that** the elastic and electrically conductive track consists of a silicone rubber loaded with an electrically conductive material which has penetrated into interstices between strands of the fabric thereby integrating and anchoring the elastic and electrically conductive track into the fabric.

2. The fabric according to claim 1, wherein the fabric further comprises a layer of an insulating material covering the elastic and electrically conductive track (1).

3. The fabric according to any one of the claims 1 to 2, wherein the fabric further comprises an electrode (2) in electrical contact with the elastic and electrically conductive track (1), the electrode configured to be placed in contact with the skin of a user.

4. The fabric according to claim 3, wherein the electrode (2) comprises a conductive fabric made of conductive fibers and non conductive fibers.

5. The fabric according to claim 3, wherein the electrode (2) comprises a layer comprising a silicone rubber and an elastic and electrically conductive material in an amount of between 5% w/w to 40% w/w, wherein the electrode is integrated into the fabric.

6. The fabric according to any one of the claims 1 to 5, wherein the electrical resistance per cm of the silicone rubber loaded with an electrically conductive material is from 50 Ω/cm to 100 kΩ/cm.

7. The fabric according to any one of the claims 1 to 5, wherein the cured temperature of the silicone rubber loaded with an electrically conductive material is from 20°C to 200 °C.

8. The fabric according to any one of the claims 1 to 7, wherein the silicone rubber comprises:
a) diorganopolysiloxane having silicon-bonded alkenyl groups;
b) organohydrogenpolysiloxanes;
c) a platinum catalyst; and
d) an electrically conductive material.

9. The fabric according to any one of the claims 1 to 8, wherein the elastic and electrically conductive material is carbon black.

10. A process for the preparation of a fabric as defined in any of the claims 1 to 9, which comprises the steps of:
a) liquid-printing a first layer of silicone rubber loaded with an amount between 5% w/w to 40% w/w of an electrically conductive material directly into the fabric, to form the at least one elastic and electrically conductive track (1);
b) pre-curing the elastic and electrically conductive track into the fabric; for up one minute at a temperature between 80°C to 200°C;
c) curing the the elastic and electrically conductive track applied into the fabric at room temperature.

11. The process according to claim 10, wherein the liquid-printing step comprises applying a pressure comprised of from 0.2 Kg/m² to 0.8 Kg/m² when printing the silicone rubber loaded with the electrically conductive material directly to the fabric.

12. The process according to claim 11, wherein the liquid-printing step comprises applying a pressure comprised of from 0.3 Kg/m² to 0.5 Kg/m² when printing the silicone rubber loaded with the electrically conductive material directly to the fabric.

13. Use of a silicone rubber loaded with an amount comprised of from 5% w/w to 40% w/w of an electrically conductive material for the preparation of the fabric according any of the claims 1 to 9.

14. A device comprising:
a) the fabric as defined in any of the claims 1 to 9,
b) an electronic instrument for receiving and collecting and/or storing and/or processing, and/or transmitting data from said fabric.

15. A garment comprising the fabric of any one of claims 1-9 or the device of claim 14.

## Patentansprüche

1. Ein Gewebe zur Erfassung physiologischer Signale umfassend mindestens eine elastische und elektrisch leitfähige Bahn mit einer Dicke von 120 µm bis 800 µm (1), wobei die elastische und elektrisch leitfähige Bahn in das Gewebe integriert ist, **dadurch gekennzeichnet, dass**
die elastische und elektrisch leitfähige Bahn aus einem mit einem elektrisch leitfähigen Material beladen Silikongummi besteht, das in die Zwischenräume zwischen den Fasern des Gewebes eingedrungen ist, wodurch die elastische und elektrisch leitfähige Bahn im Gewebe integriert und verankert ist.

2. Das Gewebe gemäß Anspruch 1, wobei das Gewebe weiter eine Schicht eines isolierenden Materials umfasst, die die elastische und elektrisch leitfähige Bahn (1) bedeckt.

3. Das Gewebe gemäß irgendeinem der Ansprüche 1 bis 2, wobei das Gewebe weiter eine Elektrode (2) in elektrischem Kontakt mit der elastischen und elektrisch leitfähigen Bahn (1) umfasst, wobei die Elektrode konfiguriert ist, in Kontakt mit der Haut eines Nutzers platziert zu werden.

4. Das Gewebe gemäß Anspruch 3, wobei die Elektrode (2) ein leitfähiges Gewebe aus leitenden Fasern und nichtleitenden Fasern umfasst.

5. Das Gewebe gemäß Anspruch 3, wobei die Elektrode (2) eine Schicht umfasst, die einen Silikongummi und ein elastisches und elektrisch leitfähiges Material in einer Menge zwischen 5 Gew.-% bis 40 Gew.-% umfasst, wobei die Elektrode ins Gewebe integriert ist.

6. Das Gewebe gemäß irgendeinem der Ansprüche 1 bis 5, wobei der elektrische Widerstand pro cm des mit einem elektrisch leitfähigen Material beladenen Silikongummis von 50 Ω/cm bis 100 kΩ/cm beträgt.

7. Das Gewebe gemäß irgendeinem der Ansprüche 1 bis 5, wobei die Härtungstemperatur des mit einem elektrisch leitfähigen Material beladenen Silikongummis von 20°C bis 200°C beträgt.

8. Das Gewebe gemäß irgendeinem der Ansprüche 1 bis 7, wobei der Silikongummi
a) Diorganopolysiloxan mit Silizium-gebundenen Alkenylgruppen;
b) Organohydrogenpolysiloxane;
c) einen Platinkatalysator; und
d) ein elektrisch leitfähiges Material
umfasst.

9. Das Gewebe gemäß irgendeinem der Ansprüche 1 bis 8, wobei das elastische und elektrisch leitfähige Material Ruß ist.

10. Ein Verfahren zur Herstellung eines wie in irgendeinem der Ansprüche 1 bis 9 definierten Gewebes, das folgende Schritte umfasst:
a) Flüssigdrucken einer ersten Schicht von Silikongummi, beladen mit einer Menge zwischen 5 Gew.-% bis 40 Gew.-% eines elektrisch leitfähigen Materials, direkt ins Gewebe, um mindestens eine elastische und elektrisch leitfähige Bahn (1) zu bilden;
b) Vorhärten der elastischen und elektrisch leitfähigen Bahn ins Gewebe über bis zu einer Minute bei einer Temperatur zwischen 80°C bis 200°C;
c) Härten der ins Gewebe eingebrachten elastischen und elektrisch leitfähigen Bahn bei Raumtemperatur.

11. Das Verfahren gemäß Anspruch 10, wobei der Flüssigdruckschritt das Anwenden eines Druckes zwischen 0,2 Kg/m² bis 0,8 Kg/m² umfasst, während der mit dem elektrisch leitfähigen Material beladene Silikongummi direkt ins Gewebe gedruckt wird.

12. Das Verfahren gemäß Anspruch 11, wobei der Flüssigdruckschritt das Anwenden eines Druckes zwischen 0,3 Kg/m² bis 0,5 Kg/m² umfasst, während der mit dem elektrisch leitfähigen Material beladene Silikongummi direkt ins Gewebe gedruckt wird.

13. Verwendung eines Silikongummis, beladen mit einer Menge zwischen 5 Gew.-% bis 40 Gew.-% eines elektrisch leitfähigen Materials, zur Herstellung des Gewebes gemäß irgendeinem der Ansprüche 1 bis 9.

14. Eine Vorrichtung umfassend:
a) das Gewebe wie in irgendeinem der Ansprüche 1 bis 9 definiert,
b) ein elektronisches Instrument zum Empfangen und Sammeln und/oder speichern und/oder Verarbeiten, und/oder Übertragen von Daten aus jenem Gewebe.

15. Ein Kleidungsstück umfassend das Gewebe aus irgendeinem der Ansprüche 1-9 oder die Vorrichtung aus Anspruch 14.

## Revendications

1. Tissu pour l'acquisition de signaux physiologiques comprenant au moins une piste élastique et électroconductrice ayant une épaisseur de 120 µm à 800 µm (1) dans lequel la piste élastique et électroconductrice est intégrée dans le tissu, **caractérisé en ce que**
la piste élastique et électroconductrice est constituée d'un caoutchouc de silicone chargé d'un matériau électroconducteur qui a pénétré dans des interstices entre des brins du tissu intégrant et ancrant ainsi la piste élastique et électroconductrice dans le tissu.

2. Tissu selon la revendication 1, dans lequel le tissu comprend en outre une couche de matériau isolant couvrant la piste élastique et électroconductrice (1).

3. Tissu selon l'une quelconque des revendications 1 et 2, dans lequel le tissu comprend en outre une électrode (2) en contact électrique avec la piste élastique et électroconductrice (1), l'électrode configurée pour être placée en contact avec la peau d'un utilisateur.

4. Tissu selon la revendication 3, dans lequel l'électrode (2) comprend un tissu conducteur composé de fibres conductrices et de fibres non conductrices.

5. Tissu selon la revendication 3, dans lequel l'électrode (2) comprend une couche comprenant un caoutchouc de silicone et un matériau élastique et électroconducteur en une quantité comprise entre 5 % p/p et 40 % p/p, dans lequel l'électrode est intégrée dans le tissu.

6. Tissu selon l'une quelconque des revendications 1 à 5, dans lequel la résistance électrique par cm du caoutchouc de silicone chargé d'un matériau électroconducteur est de 50 Ω/cm à 100 kΩ/cm.

7. Tissu selon l'une quelconque des revendications 1 à 5, dans lequel la température de durcissement du caoutchouc de silicone chargé d'un matériau électroconducteur est de 20 °C à 200 °C.

8. Tissu selon l'une quelconque des revendications 1 à 7, dans lequel le caoutchouc de silicone comprend :
a) un dioorganopolysiloxane ayant des groupes alcényle à liaison silicium ;
b) des organohydrogénopolysiloxanes ;
c) un catalyseur au platine ; et
d) un matériau électroconducteur.

9. Tissu selon l'une quelconque des revendications 1 à 8, dans lequel le matériau élastique et électroconducteur est le noir de carbone.

10. Procédé de préparation d'un tissu tel que défini dans l'une quelconque des revendications 1 à 9, qui comprend les étapes de :
a) impression liquide d'une première couche de caoutchouc de silicone chargé d'une quantité comprise entre 5 % p/p et 40 % p/p d'un matériau électroconducteur directement dans le tissu, pour former l'au moins une piste élastique et électroconductrice (1) ;
b) pré-durcissement de la piste élastique et électroconductrice dans le tissu ; pendant jusqu'à une minute à une température entre 80 °C et 200 °C ;
c) durcissement de la piste élastique et électroconductrice appliquée dans le tissu à température ambiante.

11. Procédé selon la revendication 10, dans lequel l'étape d'impression liquide comprend l'application d'une pression comprise entre 0,2 kg/m² et 0,8 kg/m² lors de l'impression du caoutchouc de silicone chargé du matériau électroconducteur directement sur le tissu.

12. Procédé selon la revendication 11, dans lequel l'étape d'impression liquide comprend l'application d'une pression comprise entre 0,3 kg/m² et 0,5 kg/m² lors de l'impression du caoutchouc de silicone chargé du matériau électroconducteur directement sur le tissu.

13. Utilisation d'un caoutchouc de silicone chargé d'une quantité comprise entre 5 % p/p et 40 % p/p d'un matériau électroconducteur pour la préparation du tissu selon l'une quelconque des revendications 1 à 9.

14. Dispositif comprenant :
a) le tissu tel que défini dans l'une quelconque des revendications 1 à 9,
b) un instrument électronique pour recevoir et collecter et/ou stocker et/ou traiter, et/ou transmettre des données dudit tissu.

15. Vêtement comprenant le tissu selon l'une quelconque des revendications 1 à 9 ou le dispositif selon la revendication 14.
